# EUROPEAN PATENT SPECIFICATION

(11) **EP 1 198 564 B1**
(45) Date of publication and mention of the grant of the patent: **14.03.2012**
(21) Application number: 00952348.1
(22) Date of filing: 01.08.2000
(51) Int. Cl.: C12N 15/01, C12N 1/20, C12P 13/04, C12P 13/08, C12R 1/15, C12R 1/13, C12R 1/19

(54) **MUTANT BACTERIAL STRAINS FOR L-LYSINE PRODUCTION**
BAKTERIENMUTANTEN ZUR L-LYSINHERSTELLUNG
SOUCHES BACTERIENNES, MUTANTES POUR LA PRODUCTION DE L-LYSINE

(30) Priority: 02.08.1999 US 146350 P
(43) Date of publication of application: 24.04.2002
(73) Proprietor: ARCHER-DANIELS-MIDLAND COMPANY, Decatur, Illinois 62525 (US)
(72) Inventor: LIAW, Hungming, J., Champaign, IL 61821 (US); EDDINGTON, John, M., Decatur, IL 62521 (US); YANG, Yueqin, Decatur, IL 62521 (US); DANCEY, Richard, Mt. Zion, IL 62549 (US); SWISHER, Stacia, Decatur, IL 62526 (US); MAO, Weiying, Decatur, IL 62521 (US)
(74) Representative: Griffin, Philippa Jane
(86) International application number: PCT/US2000/020899
(87) International publication number: WO 2001/009306

(56) References cited:
- HSIAO TZU-YIN ET AL: "Water reuse in the L-lysine fermentation process." BIOTECHNOLOGY AND BIOENGINEERING, vol. 49, no. 3, 1996, pages 341-347, XP002160959 ISSN: 0006-3592
- WEHRMANN A ET AL: "DIFFERENT MODES OF DIAMINOPIMELATE SYNTHESIS AND THEIR ROLE IN CELL WALL INTEGRITY: A STUDY WITH CORYNEBACTERIUM GLUTAMICUM" JOURNAL OF BACTERIOLOGY,WASHINGTON, DC,US, vol. 180, no. 12, June 1998 (1998-06), pages 3159-3165, XP000960667 ISSN: 0021-9193
- SONNTAG KIRSTEN ET AL: "Flux partitioning in the split pathway of lysine synthesis in Corynebacterium glutamicum: Quantification by carbon-13 and proton NMR spectroscopy." EUROPEAN JOURNAL OF BIOCHEMISTRY, vol. 213, no. 3, 1993, pages 1325-1331, XP000982370 ISSN: 0014-2956
- SANO K.; SHIIO I.: 'Microbial production of L-lysine. I. Production by auxotrophs of Brevibacterium flavum' JOURNAL OF GENERAL AND APPLIED MICROBIOLOGY vol. 13, no. 4, 1967, pages 349 - 358
- SHAH A.H. ET AL: 'Optimization of culture conditions for L-lysine fermentation by Corynebacterium glutamicum' ONLINE JOURNAL OF BIOLOGICAL SCIENCES vol. 2, no. 3, 2002, pages 151 - 156

## Description

The present invention relates to the fields of microbiology and microbial genetics. More specifically, the invention relates to novel bacterial strains, methods and processes useful for the fermentative production of amino acids, as defined in the claims.

Following the recognition that *Corynebacteria* were useful for the fermentative production of amino acids (S. Kinoshita et al., Proceedings of the International Symposium on Enzyme Chemistry 2:464-468 (1957)), the industrial production of L-lysine became an economically important industrial process. Commercial production of this essential amino acid is principally done utilizing the gram positive *Corynebacterium glutamicum, Brevibacterium flavum* and *Brevibacterium lactofermentum* (Kleemann, A., et. al., "Amino Acids," in ULLMANN'S ENCYCLOPEDIA OF INDUSTRIAL CHEMISTRY, vol. A2, pp.57-97, Weinham: VCH-Verlagsgesellschaft (1985)). These organisms presently account for the approximately 250,000 tons of L-lysine produced annually.

The efficiency of commercial production of L-lysine may be increased by the isolation of mutant bacterial strains which produce larger amounts of L-lysine. Microorganisms employed in microbial process for amino acid production are divided into 4 classes: wild-type strain, auxotrophic mutant, regulatory mutant and auxotrophic regulatory mutant (K. Nakayama et al., in Nutritional Improvement of Food and Feed Proteins, M. Friedman, ed., (1978), pp. 649-661). Mutants of *Corynebacterium* and related organisms enable inexpensive production of amino acids from cheap carbon sources, e.g., mollasses, acetic acid and ethanol, by direct fermentation. In addition, the stereospecificity of the amino acids produced by fermentation (the L isomer) makes the process advantageous compared with synthetic processes.

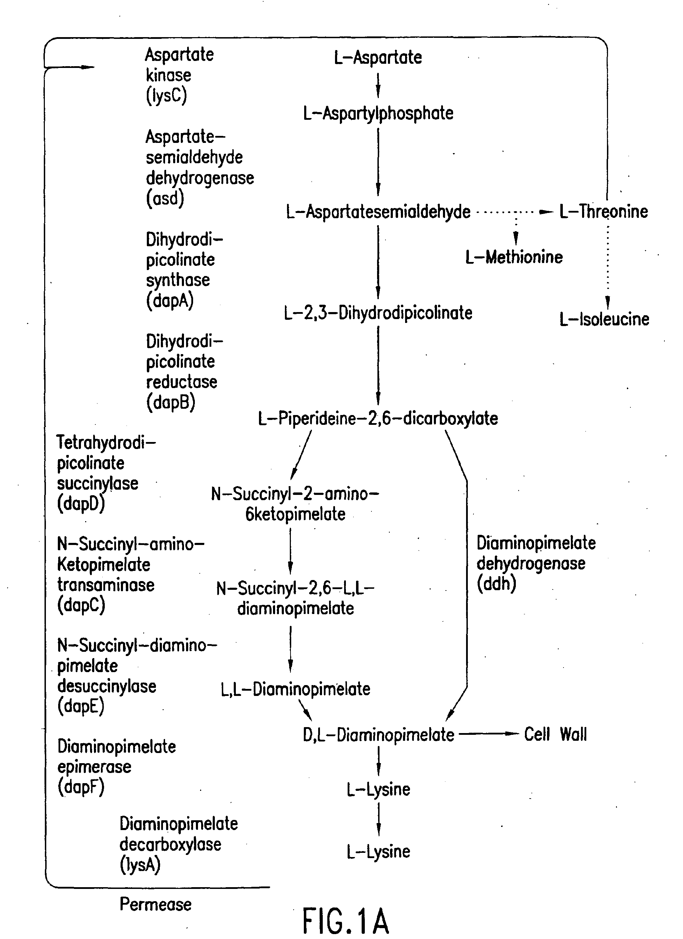

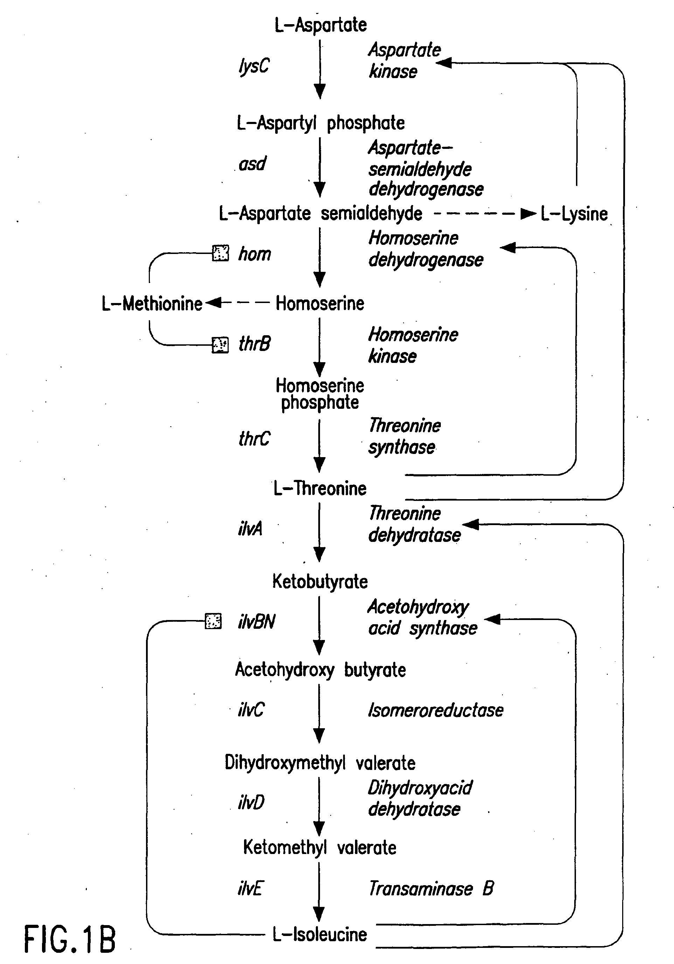

Given the economic importance of L-lysine production by the fermentive process, the biochemical pathway for lysine synthesis has been intensively investigated, ostensibly for the purpose of increasing the total amount of L-lysine produced and decreasing production costs (recently reviewed by Sahm et al., Ann. N. Y. Acad. Sci. 782:25-39 (1996)). Entry into the lysine pathway begins with L-aspartate (*see* Figure 1), which itself is produced by transamination of oxaloacetate. A special feature of *C*. *glutamicum* is its ability to convert the lysine intermediate piperidine 2,6-dicarboxylate to diaminopimelate by two different routes, i.e., by reactions involving succinylated intermediates or by the single reaction of diaminopimelate dehydrogenase. Overall, carbon flux into the pathway is regulated at two points: first, through feedback inhibition of aspartate kinase by the levels of both L-threonine and L-lysine; and second through the control of the level of dihydrodipicolinate synthase. Increased production of L-lysine may be therefore obtained in *Corynebacteria* by deregulating and increasing the activity of these two enzymes.

In addition to the biochemical pathway leading to L-lysine synthesis, recent evidence indicates that the transportation of L-lysine out of cells into the media is another factor to be considered in the development of lysine over-producing strains of *C*. *glutamicum.* Studies by Krämer and colleagues indicate that passive transport of lysine out of the cell, as the result of a leaky membrane, is not the sole explanation for lysine efflux; their data suggest a specific carrier with the following properties: (1) the transporter possesses a rather high Km value for lysine (20mM); (2) the transporter is an OH⁻ symport system (uptake systems are H⁺ antiport systems); and (3) the transporter is positively charged, and membrane potential stimulates secretion (S. Bröer and R. Krämer, Eur. J. Biochem. 202: 137-143 (1991).

Several fermentation processes utilizing various strains isolated for auxotrophic or resistance properties are known in the art for the production of L-lysine: U.S. Patent No. 2,979,439 discloses mutants requiring homoserine (or methionine and threonine); U.S. Patent No. 3,700,557 discloses mutants having a nutritional requirement for threonine, methionine, arginine, histidine, leucine, isoleucine, phenylalanine, cystine, or cysteine; U.S. Patent No. 3,707,441 discloses a mutant having a resistance to a lysine analog; U.S. Patent No. 3,687,810 discloses a mutant having both an ability to produce L-lysine and a resistance to bacitracin, penicillin G orpolymyxin; U.S. Patent No. 3,708,395 discloses mutants having a nutritional requirement for homoserine, threonine, threonine and methionine, leucine, isoleucine or mixtures thereof and a resistance to lysine, threonine, isoleucine or analogs thereof; U.S. Patent No. 3,825,472 discloses a mutant having a resistance to a lysine analog; U.S. Patent No. 4,169,763 discloses mutant strains of *Corynebacterium* that produce L-lysine and are resistant to at least one of aspartic analogs and sulfa drugs; U.S. Patent No. 5,846,790 discloses a mutant strain able to produce L-glutamic acid and L-lysine in the absence of any biotin action-surpressing agent; and U.S. Patent No. 5,650,304 discloses a strain belonging to the genus *Corynebacterium or Brevibacterium* for the production of L-lysine that is resistant to 4-N-(D-alanyl)-2,4-diamino-2,4-dideoxy-L-arabinose 2,4-dideoxy-L-arabinose or a derivative thereof.

More recent developments in the area of L-lysine fermentive production in *Corynebacteria* involve the use of molecular biology techniques to augment lysine production. The following examples are provided as being exemplary of the art: U. S. Patent Application Nos. 4,560,654 and 5,236,831 disclose an L-lysine producing mutant strain obtained by transforming a host *Corynebacterium* or *Brevibacterium* microorganism which is sensitive to S-(2-aminoethyl)-cysteine with a recombinant DNA molecule wherein a DNA fragment conferring resistance to S-(2-aminoethyl)-cysteine and lysine producing ability is inserted into a vector DNA; U. S. Patent Application No. 5,766,925 discloses a mutant strain produced by integrating a gene coding for aspartokinase, originating from *Coryneform* bacteria, with desensitized feedback inhibition by L-lysine and L-threonine, into chromosomal DNA of a *Coryneform* bacterium harboring leaky type homoserine dehydrogenase or a *Coryneform* bacterium deficient in homoserine dehydrogenase gene.

Many process designed utilizing bacterial mutant strains are designed to weaken bacterial growth and hence to enhance the yield of amino acid production through supplementation with other nutrients. Usually, mutants designed to improve the percent yield of an amino acid from substrates such as glucose will also lose their ability for vigorous growth like their wild type strains. Besides resulting in an overall decrease in amino acid yield, these mutants also require more nutrients to support their growth, which can increase the cost in the production significantly.

Hsiao Tzu-Yin et al., Biotechnology and Bioengineering, Vol. 49, No. 3, 1996, pages 341-347, describes water re-use in the L-lysine fermentation process.

Sano K. and Shiio I., J. General and Applied Microbiology, Vol. 13, No. 4, 1967, pages 349-358, describes microbial production of L-lysine; production by auxotrophs of *Brevibacterium flavum.*

Thus, there is a continuing need in the art for the development of novel amino acid producing bacterial strains that enable maximised yields of a particular amino acid at a low cost of production. In view of these problems, an alternative method comprises special mutants and media that is employed to increase the productivity and to decrease the ingredient cost.

The invention provides a method for the production of an improved lysine raffinate-resistant, L-lysine producing bacterial strain B comprising:
(a) subjecting a parental bacterial strain A to mutagenesis, wherein said parental bacterial strain A is a member of a genus selected from the group consisting of *Corynebacterium* and *Brevibacterium* that produces L-lysine;
(b) growing said mutagenized parental strain A in a medium containing 6% lysine raffinate based on ammonia sulfate content;
(c) selecting a lysine raffinate-resistant bacterial strain B,
   wherein said lysine raffinate resistant bacterial strain B has improved growth on medium containing 6% lysine raffinate, relative to bacterial strain A; and
(d) determining L-lysine production of said lysine raffinate-resistant bacterial strain B.

The specification describes a bacterial strain B that produces L-lysine, wherein said bacterial strain B is produced by a process wherein:
(a) a parental bacterial strain A having a genus selected from the group consisting of *Corynebacterium* and *Brevibacterium* is subjected to mutagenesis;
(b) the mutagenized parental strain is contacted with a medium containing at least about 1% lysine raffinate based on ammonia sulfate content;
(c) a lysine raffinate-resistant bacterial strain B is selected from said mutagenized parental strain, wherein said lysine raffinate resistant bacterial strain B has improved growth on medium containing at least about 1% lysine raffinate, relative to bacterial strain A; and
(d) amino acid production of said bacterial strain B is determined, wherein said bacterial strain B produces at least about 10 g L-lysine/liter in about 24 hours when grown in a medium containing at least about 1% lysine raffinate.

The specification further describes a process for the production of L-lysine comprising:
(a) culturing a bacterial strain B in a medium containing lysine raffinate, whereby said bacterial strain B is obtained by the following method:
   (i) selecting a parental bacterial strain A having a genus selected from the group consisting of *Corynebacterium* and *Brevibactrium* that produces L-lysine;
   (ii) subjecting said parental bacterial strain A to mutagenesis;
   (iii) selecting from said mutagenized parental strain, a lysine raffinate-resistant bacterial strain B that is able to grow in a bacterial culture medium containing 6% lysine raffinate based on ammonia sulphate content to a greater optical density than said parental bacterial strain A is able to grow in said medium, wherein said bacterial strain B is as defined in the claims; and
(b) recovering L-lysine from the culture media.

The invention relates to novel microorganisms as defined in the claims with improved lysine raffinate resistance and improved growth properties, which enables higher yields of amino acid to be produced.

A first object of the invention provides novel methods, as defined in the claims, for the production of bacterial strains with increased ability to produce L-lysine. The invention relates to a method for the production of a novel strain by way of mutagenesis of an amino acid-producing, parental bacterial strain and subsequent selection for the improved lysine raffinate resistant strains of the invention, as defined in the claims. More specifically, described are methods drawn to amino acid-producing, parental bacterial strains such as *Corynebacterium* and *Brevibacterium.* A particularly favored embodiment is drawn to a method for the production of an improved raffinate-resistant, amino acid producing bacterial strain that is *Brevibacterium* which produces L-lysine, as defined in the claims.

The specification describes novel bacterial strains with improved raffinate-resistance, improved growth characteristics and that produce larger amounts of amino acid. Bacterial strains may be produced by a process wherein a parental bacterial strain is subjected to mutagenesis and mutant progeny bacteria are selected for improved raffinate-resistance, improved growth characteristics and improved amino acid production.

Novel *Corynebacterium* or *Brevibacterium* microorganisms with improved raffinate-resistance, improved growth characteristics and improved amino acid production are described herein, such as *Brevibacterium* that produce large amounts of L-lysine. The invention provides an L-lysine producing *Corynebacterium* strain selected from ADM L63.148 (NRRL B-30059), ADM L64.132 (NRRL B-30060), ADM L69.53 (NRRL B-30061), ADM L69.74 (NRRL B-30062), and ADM L69.100 (NRRL B-30063) as defined in the claims.

The specification further provides processes for the production of L-lysine as defined in the claims, comprising the steps of (a) culturing a parent bacterium strain A in a raffinate containing medium and (b) recovering L-lysine from the culture media. The cultured bacteria of step (a) is obtained by a method in which an L-lysine-producing, parental bacterial strain A is subjected to mutagenesis and progeny are selected for improved raffinate-resistance, improved growth characteristics and improved production of L-lysine, as defined in the claims. The processes for the production of L-lysine may utilize parent strains selected from *Corynebacterium* or *Brevibacterium.* Particularly favored are processes for the production of L-lysine which utilize *Brevibacterium* that produce L-lysine.

It is to be understood that both the foregoing general description and the following detailed description are exemplary and explanatory only and are intended to provide further explanation of the invention as claimed.

### Brief Description of the Figures

Figure 1. A) A schematic presentation of the biochemical pathway leading to L-lysine production in *Corynebacterium;* B) A schematic presentation of the biochemical pathway leading to L-isoleucine production in *Corynebacterium.*

### Detailed Description of the Preferred Embodiments

### 1. Definitions

In order to provide a clear and consistent understanding of the specification and claims, including the scope to be given such terms, the following definitions are provided.
***High Yield Derivative:*** As used herein, the term refers to strain of microorganism that produces a higher yield from dextrose of a specific amino acid when compared with the parental strain from which it is derived.
***Mutation:*** As used herein, the term refers to a single base pair change, insertion or deletion in the nucleotide sequence of interest.
***Operon:*** As used herein, the term refers to a unit of bacterial gene expression and regulation, including the structural genes and regulatory elements in DNA.
***Parental Strain:*** As used herein, the term refers to a strain of microorganism subjected to some form of mutagenesis to yield the microorganism of the invention.
***Phenotype:*** As used herein, the term refers to observable physical characteristics dependent upon the genetic constitution of a microorganism.
***Raffinate:*** As used herein, the term refers to a wastestream product from an ion-exchange operation for lysine recovery. Raffinate contains a large amount of ammonia sulfate, L-lysine, other amino acids, salts, and carbohydrates such as isomaltose. Sterilization of a raffinate-containing medium using heat treatment produces amino acid derivatives and other metabolic antagonists which cause the inhibition of culture growth.
Heat sterilized raffinate-containing medium may be used to select *Brevibacterium* or *Corynebacterim* that are resistant to amino acid derivatives contained therein that inhibit culture growth; that are resistant to metabolic inhibitors contained therein that inhibit culture growth and/or that are resistant to degradation products of lysine and/or precursors to lysine contained therein that inhibit culture growth.
***Relative Growth:*** As used herein, the term refers to a measurement providing an assessment of growth by directly comparing growth of a parental strain with that of a progeny strain over a defined time period and with a defined medium.
***Mutagenesis:*** As used herein, the term refers to a process whereby a mutation is generated in DNA. With "random" mutatgenesis, the exact site of mutation is not predictable, occurring anywhere in the chromosome of the microorganism, and the mutation is brought about as a result of physical damage caused by agents such as radiation or chemical treatment.

### 2. Mutagenesis of Parental Bacterial Strains

The invention provides methods as defined in the claims for the production of bacterial strains that produce large amounts of L-lysine and have improved resistance to raffinate. Through the course of studies, it has now been found that ammonia sulfate which is required for the growth and amino acid biosynthesis may be replaced with raffinate, a wastestream product from an ion-exchange operation of lysine recovery. Raffinate contains a lot of ammonia sulfate, L-lysine, other amino acids, salts, and carbohydrates such as isomaltose. During heat treatment to sterilize the medium, however, this raffinate medium produces a lot of amino acid derivatives and other metabolic antagonists which causes the inhibition of growth for culture. To overcome this problem, a method was designed to select strains which can resist high levels of raffinate in the medium and increase their amino acid production.

Bacterial strains of the invention as defined by the claims are made by means of mutagenesis of a parental bacterial strain followed by selection of the improved raffinate-resistant phenotype. Parental strains may be selected from *Corynebacterium* and *Brevibacterium* that produce L-Iysine.

In a first embodiment, the invention provides a method for the production of an improved raffmate-resistant, L-lysine-producing, bacterial strain B comprising:
(a) subjecting a parental bacterial strain A to mutagenesis, wherein said parental bacterial strain A is a member of a genus selected from the group consisting of *Corynebacterium* and *Brevibacterium* that produces L-lysine;
(b) growing said mutagenized parental strain A in a medium containing 6% raffinate based on ammonia sulfate content;
(c) selecting a lysine raffinate-resistant bacterial strain B as defined in the claims;
(d) determining L-lysine production of said lysine raffinate-resistant bacterial strain B.

The parental strain may be mutagenized using any random mutagenesis technique known in the art, including, but not limited to, radiation and chemical procedures. Particularly preferred is random chemical mutagenesis, and most preferable is mutagenesis using a suitable agent such as N-methyl-N'-nitro-N-nitrosoguanidine (NTG).

General methods for mutagenesis and selection of novel bacterial strains are well known in the art and are described, for example, in J.H. Miller, Experiments in Molecular Genetics, Cold Spring Harbor Laboratory Press, Cold Spring Harbor, New York (1972); J.H. Miller, A Short Course in Bacterial Genetics, Cold Spring Harbor Laboratory Press, Cold Spring Harbor, New York (1992); M. Singer and P. Berg, Genes & Genomes, University Science Books, Mill Valley, California (1991); J. Sambrook, E.F. Fritsch and T. Maniatis, Molecular Cloning: A Laboratory Manual, 2d ed., Cold Spring Harbor Laboratory Press, Cold Spring Harbor, New York (1989); P.B. Kaufman et al., Handbook of Molecular and Cellular Methods in Biology and Medicine, CRC Press, Boca Raton, Florida (1995); Methods in Plant Molecular Biology and Biotechnology, B.R Glick and J.E. Thompson, eds., CRC Press, Boca Raton, Florida (1993); and P.F. Smith-Keary, Molecular Genetics of Escherichia coli, The Guilford Press, New York, NY (1989).

Strains of the invention have an improved lysine-raffinate resistant phenotype, which is determined by the concentration of raffinate, as measured by ammonium sulfate content, in the selection medium employed. Phenotype selection is done in a medium containing 6% raffinate.

The invention provides for specific novel microorganisms as defined in the claims, with improved lysine raffinate resistance and improved growth properties, which enables higher yields of amino acid to be produced. An important element or property of the methods, processes or microorganisms of the invention is related to lysine raffinate resistance.

Skilled artisans in the art of fermentative amino acid production are familiar with the term "raffinate" as used herein. However, for the purposes of more fully providing a detailed description of Applicants' invention, a definition of raffinate and a method for its production are provided.

The term "raffinate" is most closely associated with the chemical engineering field in the area of liquid-liquid extraction. The term is defined in solvent refining as "that portion of the treated liquid mixture that remains undissolved and is not removed by the selective solvent" (Dictionary of Scientific and Technical Terms, Sybil P. Parker, ed., McGraw-Hill (1989)). As used herein, the term is associated with the application of ion-exchange chromatography in the isolation of amino acids. In an analogous fashion to the process of liquid-liquid extraction, the term raffinate as used in connection with ion-exhange chromatography refers to that portion of the liquid mixture that is not selectively bound by the chromatographic resin. More specifically, in connection with the fermentative production of amino acids, the raffinate is that portion of the cell culture media that does not bind to the chromatographic column; raffinate is the broth effluent waste stream product generated during the ion-exchange chromatographic purification of an amino acid. Typically, as used herein, raffinate refers to the first waste stream product generated after the initial application of the growth media to the ion-exchange resin.

A variety of ion-exchange chromatographic methods may be utilized for the purification of amino acids. Typically, cation exchange resins are utilized for the purification of lysine. Ion-exchange chromatography may be done utilizing a fixed bed or simulated moving bed resin. For example, Van Walsem and Thompson describe a simulated moving bed technique for the isolation of lysine (Van Walsem, H. J. and Thompson, M. C., J. Biotechnology 59:127-132 (1997); U.S. Patent Nos. 4,714,767 and 5,684,190 describe the use of a fixed bed chromatographic technique for the purification of amino acids and Wolfgang and Prior utilize an annular chromatograph to achieve a continuous mode of operation in the separation of carbohydrates (Wolfgang, J. and Prior, A., Separation Science and Technology 32:71-82 (1997)). Thus, the specific chromatographic method of generating raffinate may vary, but the underlying principle defining raffinate remains constant.

For exemplary purposes only, Applicants provide in Example 5 details for the production of raffinate for use as a cell growth medium supplement. As one skilled in the art would know, raffinate may be qualitatively characterized according to the specific amino acid produced in the fermentation medium from which the raffinate is isolated; for example, raffinate may be known as lysine-raffinate when isolated from lysine fermentation medium, glycine-raffinate when isolated from glycine fermentation medium, isoleucine-raffinate when isolated from isoleucine fermentation medium, etc. It will be readily apparent to those skilled in the art that when the general term raffinate is used herein, the specific type of raffinate selected will depend upon practitioner design.

The example provided herein is exemplary for the production of raffinate, in particular for lysine-raffinate. As will be obvious to those skilled in the art, other methods may be utilized in the generation of raffinate.

### 3. Improved Raffinate Resistant Strains of the Invention

The specification describes microorganisms that have improved raffinate resistance and that produce L lysine. As one skilled in the art will know, microorganisms may selected to have improved resistance to any specific type of raffinate, for example, glycine-raffinate, valine-raffinate, isoleucine-raffinate, lysine-raffinate, etc. The microorganisms of the invention have improved resistance to lysine-raffinate.

A bacterial strain B that produces L-lysine may be produced by a method defined in the claims wherein:
(a) a parental bacterial strain A having a genus selected from the group consisting of *Corynebacterium* and *Brevibacterium* that produces L-lysine is subjected to mutagenesis;
(b) the mutagenized parental strain A is grown in a medium containing 6% raffinate based on ammonia sulfate content;
(c) a raffinate-resistant bacterial strain B is selected from said mutagenized parental strain as defined in the claims; and
(d) L-Lysine production of said raffinate-resistant bacterial strain B is determined wherein said bacterial strain B is as defined in the claims.

Selection of parental bacterial strains, mutagenesis and the selection of bacterial strains with improved lysine raffinate resistance may be done as heretofore described.

The L-lysine producing strains may be *Corynebacterium* or *Brevibacterium* that produce L-lysine.

The specification describes a *Corynebacterium sp.* strain or a *Brevibacterium* producing at least about 10g L-lysine/liter/ in 24 hours when grown in a medium containing at least about 1 % raffinate.

The invention provides an L-lysine producing *Corynebacterium* strain, wherein said strain is deposited with the NRRL under an Accession Number selected from the group consisting of NRRL B-30059, NRRL B-30060, NRRL B-30061, NRRL B-30062, and NRRL B-30063.

### 4. Amino Acid Production and Purification

The invention also relates to processes as defined in the claims for the production of L-lysine in a lysine raffinate-containing medium. Such processes involve (a) the culturing of an improved raffinate resistant bacterial strain deposited with the NRRL as defined above and (b) recovery of the L-lysine from culture media.

The process for the production of L-lysine may also comprise:
(a) culturing a bacterial strain B in a medium containing lysine raffinate, wherein said bacterial strain B is obtained by the following method:
   (i) selecting a parental bacterial strain A having a genus selected from the group consisting of *Corynebacterium* and *Brevibacterium* that produces L-lysine;
   (ii) subjecting said parental bacterial strain A to mutagenesis;
   (iii) selecting from said mutagenized parental strain, a lysine raffinate-resistant bacterial strain B that is able to grow in a bacterial culture medium containing 6% lysine raffinate based on ammonia sulfate content to a greater optical density than said parental bacterial strain A is able to grow in said medium, wherein said bacterial strain B is as defined in the claims; and
(b) recovering L-lysine from the culture media.

Selection of parental bacterial strains, mutagenesis and the selection of bacterial strains with improved raffinate resistance may be done as heretofore described.

The parental strains used in the processes of the invention are selected from the group consisting of L-lysine producing *Corynebacterium* and *Brevibacterium* microorganisms. In a most preferred embodiment of the invention, the parental strain is *Brevibacterium* that produces the amino acid L-lysine.

The processes of the invention may further vary by way of the specific method of culturing the microorganisms of the invention. Thus, a variety of fermentation techniques are known in the art which may be employed in processes of the invention drawn to the production of amino acids.

Illustrative examples of suitable carbon sources include, but are not limited to: carbohydrates, such as glucose, fructose, sucrose, starch hydrolysate, cellulose hydrolysate and molasses; organic acids, such as acetic acid, propionic acid, formic acid, malic acid, citric acid, and fumaric acid; and alcohols, such as glycerol.

Illustrative examples of suitable nitrogen sources include, but are not limited to: ammonia, including ammonia gas and aqueous ammonia; ammonium salts of inorganic or organic acids, such as ammonium chloride, ammonium phosphate, ammonium sulfate and ammonium acetate; and other nitrogen-containing, including meat extract, peptone, corn steep liquor, casein hydrolysate, soybean cake hydrolysate and yeast extract.

Generally, amino acids may be commercially produced from the invention in fermentation processes such as the batch type or of the fed-batch type. In batch type fermentations, all nutrients are added at the beginning of the fermentation. In fed-batch or extended fed-batch type fermentations one or a number of nutrients are continuously supplied to the culture, right from the beginning of the fermentation or after the culture has reached a certain age, or when the nutrient(s) which are fed were exhausted from the culture fluid. A variant of the extended batch of fed-batch type fermentation is the repeated fed-batch or fill-and-draw fermentation, where part of the contents of the fermenter is removed at some time, for instance when the fermenter is full, while feeding of a nutrient is continued. In this way a fermentation can be extended for a longer time.

Another type of fermentation, the continuous fermentation or chemostat culture, uses continuous feeding of a complete medium, while culture fluid is continuously or semi-continuously withdrawn in such a way that the volume of the broth in the fermenter remains approximately constant. A continuous fermentation can in principle be maintained for an infinite time.

In a batch fermentation an organism grows until one of the essential nutrients in the medium becomes exhausted, or until fermentation conditions become unfavorable (e.g., the pH decreases to a value inhibitory for microbial growth). In fed-batch fermentations measures are normally taken to maintain favorable growth conditions, e.g., by using pH control, and exhaustion of one or more essential nutrients is prevented by feeding these nutrient(s) to the culture. The microorganism will continue to grow, at a growth rate dictated by the rate of nutrient feed. Generally a single nutrient, very often the carbon source, will become limiting for growth. The same principle applies for a continuous fermentation, usually one nutrient in the medium feed is limiting, all other nutrients are in excess. The limiting nutrient will be present in the culture fluid at a very low concentration, often unmeasurably low. Different types of nutrient limitation can be employed. Carbon source limitation is most often used. Other examples are limitation by the nitrogen source, limitation by oxygen, limitation by a specific nutrient such as a vitamin or an amino acid (in case the microorganism is auxotrophic for such a compound), limitation by sulphur and limitation by phosphorous.

Methods for the recovery and purification of amino acids, particularly L-lysine, are well known to those skilled in the art. Typically, an amino acid may be recovered from the growth medium by cation exchange, after centrifugation and filtration to remove cells. U.S. Patent No. 5,684,190 describes the recovery of an amino acid such as L-lysine that involves (1) passage of the amino acid containing aqueous solution over a primary cation exchange resin to absorb the amino acid onto the resin at a pH lower than its isoelectric point, subsequently followed by elution of the amino acid by increasing the pH with ammonium hydroxide to produce a first solution; and (2) passage of the first solution over a secondary cation exchange resin in a similar fashion to further eliminate impurities.

Another example may be provided by U.S. Patent No. 4,714,767, which provides a process for separating basic amino acids from an aqueous solution using cation exchange resin towers in series. The process comprises repetitive adsorption and elution steps in sequence, wherein the washing water employed in the absorption and elution steps is obtained by recycling the latter portion of a liquid discharged from a first tower absorption step or elution step in a subsequent cycle.

Eluants obtained from such cation exchange isolation procedures may be concentrated by evaporation, which additionally provides for the elimination of ammonia. The amino acid may then be crystallized from solution with hydrochloric acid, producing for example L-lysine•HCl•2H₂O. After centrifugation or filtration, the isolated L-lysine crystals are dried.

### Examples

### Example 1

### Mutagenesis, Screening And Selection for Improved Raffinate Resistant Microorganisms

The lysine producing strains such a T125, L58.23, and 96T116, whose growth is inhibited by higher concentrations of raffinate, were subjected to mutagenesis, and mutants showing resistance to higher concentrations of raffinate were recovered. For mutagenesis, bacterial cultures were grown to mid-log phase in medium B (Table 1), pelleted by centrifugation and resuspended in 2 mL of filter-steriled TM buffer in a 15 ml polypropylene conical tube (Tris.HCL 6.0g/L, maleic acid 5.8 g/L, (NH₄)₂SO₄ 1.0 g/L, Ca(NO₃)₂ 5 mg/L, MgSO₄.7H₂O 0.1 g/L, FeSO₄.7H₂O 0.25 mg/L, adjusted to pH 6.0 using KOH). The 2 mL cell suspension was mixed with 50 µL of a 5.0 mg/L solution of N'-nitro-N-nitrosoguanidine (NTG), then incubated at 30°C for 30 minutes. An untreated cell suspension was similarly incubated as a control for estimating the kill rate. After incubation, 10 mL ofTM buffer was added to each tube, and the cells were pelleted by centrifugation, washed twice in TM buffer, and resuspended in 4.0 mL of 0.1 M NaH₂PO₄ (phosphate buffer) adjusted to pH 7.2 using KOH. The washed cell suspensions were further diluted in phosphate buffer, and aliquots were spread on plates of medium A (Table 1). After incubation at 30°C for 4-6 days, colonies growing on medium A agar were picked and tested for improved potential to produce L-lysine from dextrose in shaker flasks and fermentors.

### Example 2

### The Growth of Strains in Raffinate Media

For each tested strain (Table 2), 0.1 mL of frozen culture was inoculated into a 250 mL baffled flask containing 20 mL raffinate medium C (Table 1), then incubated for 18 hours at 30°C, at 240 rpm. After incubation, 50 µl of culture was removed and diluted to a ratio of 1:100 in 0.1 N HC 1 solution. The optical density (OD) of the diluted sample was measured at 660 nm with a spectrophotometer. The results are shown in Table 2. All strains with improved raffinate resistance (RF), L63.148, L64.132, L69.53, and L69.74, grew better (higher OD) than their parental strains, 108T125, L58.23, and 96T116, in the raffinate medium C.

### Example 3

### Dextrose Consumption, Growth, and Lysine Production in Shaker Flask Fermentation

For each strain, 0.1 mL of a frozen culture was inoculated into a 250 mL baffled flask containing 20 mL of seed medium C and incubated for 18 hours at 30°C, 240rpm. Two mL of seed culture were used to inoculated 20 mL of fermentation medium D in a 250 mL baffled flask. The flasks were then shaken for 24 hours at 30°C and 240 rpm. After 24 hours of fermentation, samples were removed for analysis. To measure dextrose concentrations, 100 µL of sample were removed and diluted 1:50 with deionized (DI) water and measured with a YSI biochemistry analyzer (Yellow Springs Instrument Co. Inc.). L-lysine concentrations were determined by HPLC. Optical density measurements were taken to measure growth as described in Example 2. Results are presented in Table 3; all raffinate resistant strains, L63.148, L64.132, L69.53 and L69.74, grew better, used dextrose more efficiently, and produced more L-lysine than their parent strains, 108T125, L58.23, and 96T116.

### Example 4

### Growth and L-Lysine Production in Bench Scale Fermentors

Bench scale fermentations were set up using a two stage inoculum protocol. The first stage media was composed of 50.0 g/l sucrose, 3.0 g/l K₂HPO₄, 3.0 g/l urea, 0.5 g/l MgSO₄ - 7H₂O, 30.0 g/l soy peptone, 5.0 g/l yeast extract, 0.765 mg/l biotin, 3.0 mg/l thiamine HCl, and 0.125 g/l niacinamide. A 2 liter baffled shake flask containing 500 mls of this media was inoculated with the culture and incubated at 30°C and 250 rpm for 19 hrs. At this point, 22.5 mls of the mature first culture was used to inoculate the second stage inoculum media.

The second stage inoculum was prepared with 3000 mls of medium in a 6.6 liter fermentor. The medium formulation was 20.0 g/l (db) corn steep liquor, 10.0 g/l ammonium sulfate as raffinate, 12.0 mg/l MnSO₄-H₂O, 3.0 mg/l biotin, 3.0 g/l thiamine HCl, 125 mg/l niacinamide, 0.5 mls/l antifoam, and 60 g/l dextrose, sterilized separately as a 360 g/l solution and added to the fermentor just prior to inoculation. The fermentor was operated at 32°C, 1.2 vvm air, 600 rpm, and a pH control point of 7.2. pH control was accomplished by the addition of NH₃ or NH₄OH. After 18-20 hrs the inoculum was considered mature and used to inoculate the production stage vessel.

Production stage medium was composed of 40 g/l (db) corn steep liquor, 20 g/l ammonium sulfate as raffinate, 12.0 mg/l MnSO₄-H₂O, 0.75 mls/l antifoam and 12 g/l dextrose, sterilized separately as a 250 g/l solution and added just prior to inoculation. Media formulation was based on a 2.1 liter initial volume which includes 500 mls of mature second stage broth as inoculum. Operating parameters were the following: 32°C, 2.1 vvm air, and an initial and control point pH of 7.2. pH control was again done with NH₃ or NH₄0H. Agitation was initially 600 rpm, increased to 700 rpm at 9 hrs culture time and 900 rpm at 19 hrs culture time. The fermentation was fed on demand, as indicated by pH increases due to dextrose depletion, a mixture of dextrose and ammonium sulfate. The feed was prepared by sterilizing separately 2310 g dextrose + 800 mls water and a volume of raffinate containing a total of 176 g of ammonium sulfate, then combining the two solutions upon cooling to ambient temperature. Total fermentation time was 48 hrs. The vessel size was the same as that used for the second stage inoculum development.

Results of an experiment comparing the parent strain to the above described isolates in bench scale fermentation are presented in Table 4.

### Example 5 (For illustration)

### Production of Raffinate

As previously described, raffinate may be may be qualitatively characterized according to the specific amino acid produced in the fermentation medium from which the raffinate is isolated. The example provided herein is for the production of lysine-raffinate. However, one skilled in the art would know, other types of raffinate, e.g., valine- or isoleucine-raffinate, etc., may be similarly produced by simply starting with the appropriate fermentation medium, e.g., valine or isoleucine fermentation medium, etc.

As a first step in the production of lysine-raffinate, lysine fermentation growth medium is diluted to a lysine concentration of 65.5 g/l. After ultrafiltration to generate a permeate with a lysine concentration of 40.3 g/l, the permeate is then concentrated to 123 g/l lysine with a total dry solids concentration of 207 g/l.

The permeate concentrate is then fed into a chromatographic separation system, for example I-SEP or C-SEP produced by Advanced Separation Technologies Incorporated (St. Petersburg, FL). Ion exchange chromatographic separation systems are commonly known in the art, as exemplified by U.S. Patent Nos. 4,808,317 and 4,764,276, which are incorporated herein by reference. The waste effluent obtained therefrom is considered the "dilute lysine-raffinate" solution. The dilute lysine-raffinate solution has a pH of 5.1 and it contains 34.3 g/l ammonium sulfate and 2.8 g/l lysine with a total solids level 67 g/l.

The dilute lysine-raffinate solution is concentrated to 295 g/l total solids. Quantitated components of this "concentrated lysine-raffinate" solution include the following: 137.9 g/l ammonium sulfate, 14.8 g/l lysine, 8.7 g/l valine, 8.1 g/l alanine, 2.4 g/l lactic acid and 2.2 g/l acetic acid. This concentrated lysine-raffinate solution is used in media preparation.

### TABLES

The following tables are referenced in the Examples section.

**Table 1**

| **Media Employed in Examples 1, 2, and 3** | | | | |
|---|---|---|---|---|
| Ingredients (amount/L) | A | B | C | D |
| Glucose | 20 g | | 30 g | 68 g |
| Sucrose | | 50 g | | |
| L-Alanine | 0.5 g | 0.5 g | | |
| L-Methionine | 0.5 g | 0.5 g | | |
| L-Threonine | 0.25 g | 0.25 g | | |
| Biotin | 0.05 mg | 0.756 mg | 0.003 g | 0.405 mg |
| Thiamine | 0.2 mg | 0.003 g | 0.003 g | |
| Niacinamide | 0.05 g | 0.125 g | 0.125g | |
| Polypeptone Peptone (BBL) | | 20 g | | |
| Beef Extract (BBL) | | 5 g | | |
| Corn Steep Liquor¹ | | | 20 g | |
| Raffinate² | 60 g | | 10 g | 40 g |
| Urea | 2.5 g | 3 g | | 50 g |
| Amonia Sulfate | 10 g | | | |
| K₂HPO₄ | | 3 g | | |
| KH₂PO₄ | 1 g | | | |
| MgSO₄.7H₂O | 0.4 g | 0.5 g | | |
| MnSO₄.H₂O | 0.01 g | | 0.01 g | 0.01 g |
| NaCl | 1 g | | | |
| FeSO₄.7H₂O | 0.01 g | | | |
| CaCO₃ | | | 50 g | 50 g |
| Agar | 15 g | | | |
| pH (before autoclave) | 7.2 | 7.3 | 7.4 | 7.4 |

| | | | | |
|---|---|---|---|---|
| 1. The amount of corn steep liquor is expressed as grams of dried solids per liter of medium. 2. The amount of raffinate is expressed as grams of ammonia sulfate per liter of medium. | | | | |

**Table 2**

| **The Growth of Strains in Medium C Containing Raffinate** | | | | | | | |
|---|---|---|---|---|---|---|---|
| Strain | 108T125 | L63.148 | L58.23 | L64.132 | 96T116 | L69.53 | L69.74 |
| Type | Wild¹ | RF² | Wild | RF | Wild | RF | RF |
| OD₆₆₀ | 15.9 | 27.4 | 27.1 | 34.5 | 22.2 | 31.6 | 30.3 |

| | | | | | | | |
|---|---|---|---|---|---|---|---|
| 1. Strains 108T125, L58.23, and 96T116 are parent and wild type strains used to generate the improved raffinate resistant strains of the invention. 2. Strains L63.148, L64.132, L69.53, and L69.74 are improved raffinate resistant (RF) strains derived from their wild type parental strains as described. | | | | | | | |

**Table 3**

| **The Dextrose Consumption (Dex), Growth (OD₆₆₀), and Lysine Production (Lys) of Strains in 24 hr Shaker Flask Fermentation in Medium D** | | | | | | | |
|---|---|---|---|---|---|---|---|
| Strain | 108T125 | L63.148 | L58.23 | L64.132 | 96T116 | L69.53 | L69.74 |
| Type | Wild | RF | Wild | RF | Wild | RF | RF |
| Dex, g/L | 25.9 | 66.7 | 40.6 | 68.8 | 45.8 | 78.8 | 76.6 |
| OD₆₆₀ | 20.5 | 43.2 | 26.3 | 47.9 | 30.5 | 47.4 | 42.8 |
| Lys, g/L | 9.4 | 18.8 | 14.1 | 23.3 | 15.5 | 24.6 | 23.2 |

**Table 4**

| **Parent and Progeny Comparison of Growth (OD660) and L-lysine Production in 6.6 1 fermentors** | | | | |
|---|---|---|---|---|
| Strain | | OD @ 660nm | Total Product¹ | g lysine/1/hr² |
| 96T116 | Wild | 83.8 | 583 g | 5.78 |
| L69.53 | RF | 112.5 | 776 g | 7.70 |
| L69.74 | RF | 122.4 | 807 g | 8.01 |
| L69.100 | RF | 93.3 | 745 g | 7.48 |

| | | | | |
|---|---|---|---|---|
| 1. Total Product denotes total grams of lysine in the fermentor at harvest. 2. Calculation based on the initial 2.1 liter volume. | | | | |

## Claims

1. A method for the production of an improved lysine raffinate-resistant, L-lysine producing bacterial strain B comprising:
(a) subjecting a parental bacterial strain A to mutagenesis, wherein said parental bacterial strain A is a member of a genus selected from the group consisting of *Corynebacterium* and *Brevibacterium* that produces L-lysine;
(b) growing said mutagenized parental strain A in a medium containing 6% lysine raffinate based on ammonia sulfate content;
(c) selecting a lysine raffinate-resistant bacterial strain B, wherein said lysine raffinate resistant bacterial strain B has improved growth on medium containing 6% lysine raffinate, relative to bacterial strain A; and
(d) determining L-lysine production of said lysine raffinate-resistant bacterial strain B.

2. The method of Claim 1, wherein said parental bacterial strain is subjected to random chemical mutagenesis.

3. The method of Claim 1, wherein said parental bacterial strain A is *Corynebacterium sp*., producing L-lysine.

4. An L-lysine producing *Corynebacterium sp.* strain, wherein said strain is deposited with the NRRL under an Accession Number selected from the group consisting of:
(a) NRRL B-30059;
(b) NRRL B-30060;
(c) NRRL B-30061;
(d) NRRL B-30062; and
(e) NRRL B-30063.

5. The strain of Claim 4, wherein said strain is deposited as Accession Number NRRL B-30059.

6. The strain of Claim 4, wherein said strain is deposited as Accession Number NRRL B-30060.

7. The strain of Claim 4, wherein said strain is deposited as Accession Number NRRL B-30061.

8. The strain of Claim 4, wherein said strain is deposited as Accession Number NRRL B-30062.

9. The strain of Claim 4, wherein said strain is deposited as Accession Number NRRL B-30063.

10. A process for the production of L-lysine comprising:
(a) culturing a *Corynebacterium sp.* strain deposited with the NRRL under an Accession Number selected from the group consisting of:
(a) NRRL B-30059;
(a) NRRL B-30060;
(c) NRRL B-30061;
(d) NRRL B-30062; and
(e) NRRL B-30063
in a medium containing lysine raffinate; and
(b) recovering L-lysine from the culture media.

11. The process of Claim 10, wherein the amount of L-lysine produced by said bacterial strain is at least about 10 g/l L-lysine in 24 hours.

## Patentansprüche

1. Verfahren für die Erzeugung eines verbesserten lysinraffinatresistenten L-Lysinproduzierenden Bakterienstamms B, umfassend:
(a) Unterziehen eines bakteriellen Parentalstamms A einer Mutagenese, wobei der bakterielle Parentalstamm A ein Mitglied einer Gattung ausgewählt aus der Gruppe bestehend aus *Corynebacterium* und *Brevibacterium* ist, der L-Lysin produziert;
(b) Kultivieren des mutagenisierten Parentalstamms A in einem Medium, das basierend auf dem Ammoniumsulfatgehalt 6 % Lysinraffinat enthält;
(c) Selektieren eines lysinraffinatresistenten Bakterienstamms B, wobei der lysinraffinatresistente Bakterienstamm B relativ zum Bakterienstamm A auf Medium enthaltend 6 % Lysinraffinat ein verbessertes Wachstum aufweist; und
(d) Bestimmen der L-Lysin-Produktion des lysinraffinatresistenten Bakterienstamms B.

2. Verfahren nach Anspruch 1, wobei der bakterielle Parentalstamm einer chemischen Zufallsmutagenese unterzogen wird.

3. Verfahren nach Anspruch 1, wobei der bakterielle Parentalstamm A *Corynebacterium sp.* ist, der L-Lysin produziert.

4. L-Lysin-produzierender *Corynebacterium sp*.-Stamm, wobei der Stamm unter der Zugangsnummer ausgewählt aus der Gruppe bestehend aus
(a) NRRL B-30059;
(b) NRRL B-30060;
(c) NRRL B-30061;
(d) NRRL B-30062; und
(e) NRRL B-30063
bei der NRRL hinterlegt ist.

5. Stamm nach Anspruch 4, wobei der Stamm unter der Zugangsnummer NRRL B-30059 hinterlegt ist.

6. Stamm nach Anspruch 4, wobei der Stamm unter der Zugangsnummer NRRL B-30060 hinterlegt ist.

7. Stamm nach Anspruch 4, wobei der Stamm unter der Zugangsnummer NRRL B-30061 hinterlegt ist.

8. Stamm nach Anspruch 4, wobei der Stamm unter der Zugangsnummer NRRL B-30062 hinterlegt ist.

9. Stamm nach Anspruch 4, wobei der Stamm unter der Zugangsnummer NRRL B-30063 hinterlegt ist.

10. Verfahren für die Herstellung von L-Lysin, umfassend:
(a) Kultivieren eines *Corynebacterium sp*.-Stammes, der bei der NRRL unter einer Zugangsnummer hinterlegt ist, die ausgewählt ist aus der Gruppe bestehend aus:
(a) NRRL B-30059;
(b) NRRL B-30060;
(c) NRRL B-30061;
(d) NRRL B-30062; und
(e) NRRL B-30063,
in einem Medium enthaltend Lysinraffinat; und
(b) Isolieren von L-Lysin aus dem Kulturmedium.

11. Verfahren nach Anspruch 10, wobei die L-Lysin-Menge, die von dem Bakterienstamm produziert wird, wenigstens ungefähr 10 g/l L-Lysin in 24 Stunden beträgt.

## Revendications

1. Procédé de production d'une souche bactérienne B productrice de L-lysine améliorée, résistante au raffinat de lysine, comprenant
(a) la soumission d'une souche bactérienne mère A à une mutagenèse, ladite souche bactérienne mère A étant un membre d'un genre choisi dans le groupe constitué de *Corynebacterium* et *Brevibacterium* qui produit de la L-lysine;
(b) la culture de ladite souche mère A mutée dans un milieu contenant 6 % de raffinat de lysine par rapport à la teneur en sulfate d'ammonium;
(c) la sélection d'une souche bactérienne B résistante au raffinat de lysine, ladite souche bactérienne B résistante au raffinat de lysine ayant par rapport à la souche bactérienne A une croissance améliorée dans un milieu contenant 6 % de raffinat de lysine; et
(d) la détermination de la production de L-lysine de ladite souche bactérienne B résistante au raffinat de lysine.

2. Procédé selon la revendication 1, dans lequel ladite souche bactérienne mère est soumise à une mutagenèse chimique aléatoire.

3. Procédé selon la revendication 1, dans lequel ladite souche bactérienne mère A est *Corynebacterium sp.* productrice de L-lysine.

4. Souche de *Corynebacterium sp.* productrice de L-lysine, ladite souche étant déposée auprès de la NRRL sous un numéro d'accession choisi dans le groupe constitué de:
(a) NRRL B-30059;
(b) NRRL B-30060;
(c) NRRLB-30061;
(d) NRRL B-30062; et
(e) NRRL B-30063.

5. Souche selon la revendication 4, ladite souche étant déposée sous le numéro d'accession NRRL B-30059.

6. Souche selon la revendication 4, ladite souche étant déposée sous le numéro d'accession NRRL B-30060.

7. Souche selon la revendication 4, ladite souche étant déposée sous le numéro d'accession NRRL B-30061.

8. Souche selon la revendication 4, ladite souche étant déposée sous le numéro d'accession NRRL B-30062.

9. Souche selon la revendication 4, ladite souche étant déposée sous le numéro d'accession NRRL B-30063.

10. Procédé de production de L-lysine, comprenant
(a) la culture d'une souche de *Corynebacterium sp.* déposée auprès de la NRLL sous un numéro d'accession choisi dans le groupe constitué de:
(a) NRRL B-30059;
(b) NRRL B-30060;
(c) NRRLB-30061;
(d) NRRL B-30062; et
(e) NRRL B-30063
dans un milieu contenant du raffinat de lysine; et
(b) la récupération de L-lysine à partir du milieu de culture.

11. Procédé selon la revendication 10, dans lequel la quantité de L-lysine produite par ladite souche bactérienne est d'au moins environ 10 g/l de L-lysine en 24 heures.
